Europäisches Patentamt

(19) European Patent Office

Office européen des brevets

(11) Publication number: **0 128 709**
**B1**

(12) # EUROPEAN PATENT SPECIFICATION

(45) Date of publication of patent specification: **02.11.88**    (51) Int. Cl.⁴: **C 07 C 55/02, C 07 C 51/42**

(21) Application number: **84303684.9**

(22) Date of filing: **01.06.84**

(54) Semi-refined mixed dicarboxylic acids.

<table>
<tr><td>

(30) Priority: **07.06.83 CA 429826**

(43) Date of publication of application:
**19.12.84 Bulletin 84/51**

(45) Publication of the grant of the patent:
**02.11.88 Bulletin 88/44**

(84) Designated Contracting States:
**DE FR GB**

(56) References cited:
**DE-A-2 051 320**
**FR-A-2 111 380**
**US-A-4 014 903**

</td><td>

(73) Proprietor: **DU PONT CANADA INC.**
**Box 2200 Streetsville Postal Station**
**Mississauga Ontario L5M 2H3 (CA)**

(72) Inventor: **Leboeuf, Christiaan**
**33 Montgomery Blvd Apt. "B"**
**Kingston OntarioK7M 3N7 (CA)**

(74) Representative: **Ellis, Edward Lovell et al**
**MEWBURN ELLIS & CO. 2/3 Cursitor Street**
**London EC4A 1BQ (GB)**

</td></tr>
</table>

Courier Press, Leamington Spa, England.

## Description

This invention relates to a process for the recovery of semi-refined mixed dicarboxylic acids and more particularly to the recovery of mixed adipic, glutaric and succinic acids from a mother liquor, or a partially evaporated mother liquor, containing said dicarboxylic acids together with catalyst residues and impurities.

Production of aliphatic dicarboxylic acids by the oxidation of cycloparaffins is of considerable commercial importance because of the availability of cycloparaffins and the widespread use of such acids in the manufacture of synthetic resins. An example is the production of adipic acid, an intermediate for nylon 6, 6, by the oxidation of cyclohexane. In the manufacture of adipic acid, it has been found preferable to oxidize cyclohexane in a two step oxidation process, i.e. to oxidize cyclohexane with air to a mixture containing cyclohexanol and cyclohexanone and to thereafter oxidize at least a portion of that mixture using nitric acid to a mixture of mono- and di-carboxylic acids in an aqueous nitric acid solution, for example, as described by C. H. Hamblet and A. McAlevy in U.S. 2 557 282, which issued 1951 June 19. Copper and/or vanadium catalysts may be used in the nitric acid oxidation step. After crystallization of a portion of the adipic acid from this mixture, at least a part of the mother liquor remaining is subjected to evaporating conditions to reduce the nitric acid concentration of the liquor. Further crystallization of the adipic acid is then effected. The mother liquor from the second crystallization contains adipic, glutaric and succinic acids together with catalyst residues and impurities including nitric acid. Separation of additional amounts of adipic acid from the mother liquor may be difficult because of co-crystallization of succinic acid.

Various processes have been proposed heretofore to recover some of the useful components from this mother liquor or from the mixture remaining after evaporation to reduce the nitric acid concentration. Canadian Patent 707 340 of Gilby, Hoberg and Phibbs, issued 1956 April 06, describes the separation by distillation of succinic and glutaric acids from adipic acid as anhydrides. Canadian Patent 955 876 of Adamek, issued 1974 October 08, describes a process in which a mixture of adipic, succinic and glutatic acids is treated with ammonia in less than the stoichiometric amount required for complete ammoniation. The imides of the succinic and glutaric acids are then separated by distillation from the adipic acid. In another known process a mixture of adipic, succinic and glutatic acids is reacted with methanol and the mixed esters so produced are distilled overhead as a useful product.

Some of the above prior art processes are expensive to operate. In another process there is insufficient demand for the product to utilize all of the mother liquor which is available.

In a process disclosed in DE—A—2 051 320, a crude mixture of dicarboxylic acids is purified by distilling off nitric acid and water, optionally heat-treating at 180°C for two hours and distilling the acids at a temperature of 170—240°C and pressure of 1—20 mm Hg (133 Pa—2.7 kPa). No mention is made of colour reduction in the products.

It has been established that a market could exist for a low cost semi-refined mixture of adipic, glutaric and succinic acids, provided that said mixture when reacted with ethylene glycol and/or diethylene glycol, produces a low molecular weight polyester having an acceptable colour level e.g. not more than about 5 on the so-called Gardner scale i.e. a light brown colour. In the Gardner Varnish Colour System of the Institute of Paint and Varnish Research (ASTM D 154) the larger the number, the darker the colour. One, the lowest number on the Gardner scale, corresponds to a colour of 270 in the Platinum-Cobalt (APHA) system (ASTM D 1209).

It has now been found that such a semi-refined mixture of adipic, glutaric and succinic acids may be produced from an aqueous mixture of such acids, catalyst residues and impurities, by dehydration followed by the evaporation of the mixed acids from the dehydrated mixture in an evaporating means, provided that prior to the dehydration step a reducing agent selected from the group consisting of hypophosphorous acid and sodium hypophosphite is added to the aqueous mixture.

Accordingly the present invention provides a process for reciving a semi-refined mixture of adipic, gluratic and succinic acids from an aqueous mixture derived from a process for the oxidation of cyclohexane to dicarboxylic acids, said mixture comprising said acids together with catalyst residues and impurities, the process comprising the steps of:

(a) adding to the aqueous mixture a reducing agent selected from the group consisting of hypophosphorous acid and sodium hypophosphite;

(b) concentrating the resulting aqueous mixture by the removal of water to produce a substantially dehydrated mixture; and

(c) feeding the substantially dehydrated mixture to an evaporating means and evaporating therefrom a mixture of adipic, glutaric and succinic acids.

In an embodiment of the process of present invention the recorved semi-refined mixture of adipic, glutaric and succinic acids is capable of being reacted with ethylene glycol and/or diethylene glycol to produce a low molecular weight polyester having a colour level of not more than about 5 on the Gardner scale.

In another embodiment of the process of present imvention, the evaporating step (c) is carried out in a wiped-film evaporator.

In yet another embodiment of the process of the present invention, both the evaporating step (c) and the concentrating step (b) are carried out in a wiped-film evaporator.

Yet another embodiment of the process of the present invention includes the additional steps of:

(d) condensing the mixture of adipic, glutaric and succinic acids evaporated from the evaporating means in step (c), as a molten material; and

(e) feeding the molten material to a flaker to produce a product in flake form.

In a further embodiment of the process of the present invention, the reducing agent is hypophosphorous acid present in an amount of 0.5 to 2.0 percent by weight of the aqueous mixture.

In a still further embodiment of the process of the present invention, the reducing agent is sodium hypophosphite present in an amount 1.0 to 4.0 percent by weight of the aqueous mixture.

The present invention also provides a semi-refined mixture of adipic, glutaric and succinic acids, said mixture being capable of being reacted with ethylene glycol and/or diethylene glycol to produce a low molecular weight polyester having a colour level of not more that about 5 on the Gardner scale, said mixture having been produced from an aqueous mixture derived from a process for the oxidation of cyclohexane to dicarboxylic acids, said mixture comprising said acids together with catalyst residues and impurities by a process comprising the steps of:

(a) adding to the aqueous mixture a reducing agent selected from the group consisting of hypophosphorous acid and sodium hypophosphite;

(b) concentrating the resulting aqueous mixture by the removal of water to produce a substantially dehydrated mixture; and

(c) feeding the substantially dehydrated mixture to an evaporating means and evaporating therefrom a mixture of adipic, glutaric and succinic acids.

In another embodiment of the semi-refined mixture of adipic, glutaric and succinic acids of the present invention, said mixture contains less than 500 parts per million (by weight) of phosphorus.

As hereinbefore described the present invention provides a process for the recovery of a semi-refined mixture of adipic, glutaric and succinic acids from an aqueous mixture of such acids together with impurities. In a preferred embodiment of the present invention the aqueous mixture of dicarboxylic acids is in the form of a partially concentrated mother liquor. The mother liquor may be obtained as waste from the adipic acid recovery steps of a process for the manufacture of adipic acid by the oxidation of cyclohexane. Such mother liquors may contain monobasic acids, catalyst residues from a previous stage of the process, and other impurities, for example nitric acid and nitrogen-containing colour-forming materials, for example, nitroaliphatic acids.

The aqueous mixture of dicarboxylic acids obtained as hereinbefore described may, according to the present invention, be first treated with a suitable reducing agent and then fed to a concentrating means, for example, an evaporator, preferably a wiped-film evaporator. In the concentrating means the mixture may be substantially dehydrated by the removal of water and nitric acid.

The substantially dehydrated mixture may then be fed to an evaporating means, especially a wiped-film evaporator, for the evaporation therefrom of a mixture of adipic, glutaric and succinic acids. The mixture of adipic, glutaric and succinic acids evaporated from the evaporating means, if desired, may be condensed as a molten material and fed to a flaker to produce a product in flake form. The tails from the evaporating means, which contain residual dicarboxylic acids, catalyst residues and high boiling impurities, may be purged to waste, for example, by burning.

It will be appreciated that if a storage tank is provided for the substantially dehydrated mixture from the concentrating means, a single wiped-film evaporator may be employed, in turn, as the concentrating means and as the evaporating means.

The reducing agent may be added in a feed storage tank or it may be added directly to the feed stream to the concentrating means. The reducing agent is preferably selected from the group consisting of hypophosphorous acid and sodium hypophosphite. If the reducing agent is hypophosphorous acid it preferably should be present in an amount of 0.5 to 2.0 percent by weight of the aqueous mixture fed to the concentrating means. If the reducing agent is sodium hypophosphite it preferably should be present in amount of 1.0 to 4.0 percent by weight of the aqueous mixture.

The preferred wall temperature range for the concentrating means, for example, a wiped-film evaporator, is from 150 to 190°C and in particular 160 to 180°C. The preferred operating pressure range for the concentrating means, for example a wiped-film evaporator, is from 150 to 600 mm of mercury absolute (20—80 kPa) and in particular from 200 to 400 mm of mercury absolute (26.7—53.3 kPa).

The preferred wall temperature range for the evaporating means, for example a wiped-film evaporator, is from 200 to 260°C and in particular, from 215 to 250°C. The preferred operating pressure range for the evaporating means, for example a wiped-film evaporator, is from 5 to 25 mm of mercury absolute (0.67—3.33 kPa) and in particular from 5 to 15 mm of mercury absolute (0.67—2.0 kPa), the lower limit usually being determined by the limitations of the process equipment.

In the evaporation step, it is preferred that from 80 to 95 percent by weight of the substantially dehydrated mixture be evaporated as adipic, glutaric and succinic acid.

The present invention may be further illustrated by the following examples:

Example 1

0.95 ml. of 50% by weight hypophosphorous acid was added to 118 g of an aqueous mixture having the following approximate composition: adipic acid 11% by weight, glutaric acid 50% by weight, succinic acid 19% by weight, water 20% by weight, nitric acid, 0.1% by weight, copper 100

parts per million and vanadium 350 parts per million. The hypophosphorous acid was present in the amount 0.5% by weight of the aqueous mixture. The resulting mixture was maintained for one hour at a temperature of 90°C and was then dehydrated in a shallow dish for 10 minutes in an oven at a temperature of 95°C and a pressure of 300 mm Hg absolute (40 kPa).

The dehydrated mixture was then evaporated in a tubular stainless steel furnace at a pressure of 21 mm Hg absolute (2.8 kPa) and a wall temperature of 230—254°C. The evaporated mixed acid product was obtained at a yield of 95% and contained 320 parts per million (by weight) phosphorus (approximately 10% of the total added).

A low molecular weight polyester was made by mixing 15 g of the product with 13 g of clear, colourless diethylene glycol (histological grade) in a test tube and then heating in an oil bath for one hour at a temperature of 190°C+2°C. The colour of the polyester was 5 on the scale of the Gardner Varnish Colour System as discussed hereinbefore.

Example 2

Sodium hypophosphite was added to 125 g of the aqueous mixture of dibasic acids as described in Example 1. The sodium hypophosphite was present in the amount of 1.0% by weight of the aqueous mixture. The resulting mixture was maintained for one hour at a temperature of 90°C and was then dehydrated and evaporated as described in Example 1. The mixed acid product obtained, at a yield of 90%, contained 372 parts per million (by weight) phosphorus (approximately 9% of the total added). A low molecular weight polyester was prepared from the product in the same manner as described in Example 1. The colour of the polyester was 5 on the Gardner scale.

Example 3

Hypophosphorous acid was added to 159 g of the aqueous mixture of dibasic acids described in Example 1. The hypophosphorous acid was present in the amount of 1% by weight of the aqueous mixture. The resulting mixture was maintained for 90 minutes at a temperature of 90°C and was then dehydrated and evaporated as described in Example 1. The mixed acid product obtained, at a yield of 89%, contained 330 parts per million (by weight) phosphorus (approximately 5% of the total added). A low molecular weight polyester was prepared from the product in the same manner as described in Example 1. The colour of the polyester was 3.5 on the Gardner scale.

Example 4

Sodium hypophosphite was added to 166 g of the aqueous mixture of dibasic acids described in Example 1. The sodium hypophosphite was present in an amount equivalent to 1.6% by weight of the aqueous mixture. The resulting mixture was maintained for 90 minutes at a temperature of 90°C and was then dehydrated and evaporated as described in Example 1. The mixed acid product obtained, at a yield of 85%, contained 150 parts per million (by weight) phosphorus (approximately 2% of the total added). A low molecular weight polyester was prepared from the product in the same manner as described in Example 1. The colour of the polyester was 3.5 on the Gardner scale.

Example 5

For comparative purposes a test was carried out similar to Examples 1 to 4 except that no hypophosphorous acid or sodium hypophosphite was added.

120 g of an aqueous mixture of dibasic acids of composition as described in Example 1 was dehydrated in a shallow dish for approximately 15 minutes in an oven at a temperature of 95°C and a pressure of 300 mm Hg absolute (40 kPa). The dehydrated mixture was then evaporated in a tubular stainess steel furnace at pressure of 20 mm Hg absolute (2.67 kPa) and a wall temperature of 232—248°C). The evaporated mixed acid product was obtained at a yield of 93%. The product was off-white in colour and contained less than one part per million (by weight) of copper and vanadium. A low molecular weight polyester was prepared from the product in the same manner as described in Example 1. Te colour of the polyester was 12 (brown) on the Gardner colour scale.

**Claims**

1. A process for recovering a semi-refined mixture of adipic, glutaric and succinic acids from an aqueous mixture derived from a process for the oxidation of cyclohexane to dicarboxylic acids, said mixture comprising said acids together with catalyst residues and impurities, the process comprising the steps of:

   (a) adding to the aqueous mixture a reducing agent selected from hypophosphorous acid and sodium hypophosphite;

   (b) concentrating the resulting aqueous mixture by the removal of water to produce a substantially dehydrated mixture; and

   (c) feeding the substantially dehydrated mixture to an evaporating means and evaporating therefrom a mixture of adipic, glutaric and succinic acids.

2. The process according to Claim 1 wherein the recovered semi-refined mixture of adipic, glutaric and succinic acids is capable of being reacted with ethylene glycol and/or diethylene glycol to produce a low molecular weight polyester having a colour level of not more than about 5 on the Gardner scale.

3. The process according to Claim 1 or Claim 2, wherein the evaporating step (c) is carried out in a wiped-film evaporator.

4. The process according to any preceding claim, wherein the concentrating step (b) is carried out in a wiped-film evaporator.

5. The process according to any preceding claim, including the additional steps of:

(d) condensing the mixture of adipic, glutaric and succinic acids evaporated from the evaporating means in step (c) as a molten material; and

(e) feeding the molten material to a flaker to produce a product in flake form.

6. The process according to any preceding claim, wherein the reducing agent is hypophosphorous acid present, in an amount of 0.5 to 2.0 percent by weight of the aqueous mixture.

7. The process according to any one of Claims 1 to 5, wherein the reducing agent is sodium hypophosphite present in an amount of 1.0 to 4.0 percent by weight of the aqueous mixture.

8. The process of any preceding claim, wherein 80 to 95 percent by weight of the substantially dehydrated mixture is evaporated as a mixture of adipic, glutaric and succinic acids.

**Patentansprüche**

1. Verfahren zur Abtrennung einer halb-raffinierten Mischung von Adipinsäure, Glutarsäure und Bernsteinsäure von einem wäßrigen Gemisch, das aus einem Verfahren zur Oxidation von Cyclohexan zu Dicarbonsäuren stammt, das diese Säuren zusammen mit Katalysatorresten und Verunreinigungen enthält, das die folgenden Stufen umfaßt:

(a) Hinzufügen eines Reduktionsmittels, ausgewählt aus Hypophosphoriger Säure und Natriumhypophosphit, zu dem wäßrigen Gemisch;

(b) Konzentriern des resultierenden wäßrigen Gemisches durch Entfernung von Wasser unter Bildung eines im wesentlichen dehydratisierten Gemisches; und

(c) Einführen des im wesentlichen dehydratisierten Gemisches in eine Verdampfungseinrichtung und Verdampfen einer Mischung von Adipinsäure, Glutarsäure und Bernsteinsäure daraus.

2. Verfahren nach Anspruch 1, worin die abgetrennte halbraffinierte Mischung von Adipinsäure, Glutarsäure und Bernsteinsäure mit Ethylenglycol und/oder Diethylenglycol umgesetzt werden kann zur Herstellung eines Polyesters mit einem niedrigen Molekulargewicht, der einen Farbwert von nicht mehr als etwa 5 auf der Gardner-Skala aufweist.

3. Verfahren nach Anspruch 1 oder 2, worin die Verdampfungsstufe (c) in einem Abstreif-Film-Verdampfer durchgeführt wird.

4. Verfahren nach einem der vorhergehenden Ansprüche, worin die Konzentrationsstufe (b) in einem Abstreif-Film-Verdampfer durchgeführt wird.

5. Verfahren nach einem der vorhergehenden Ansprüche, das die zusätzlichen Stufen umfaßt:

(d) Kondensieren der aus der Verdampfungseinrichtung in der Stufe (c) verdampften Mischung von Adipinsäure, Glutarsäure und Bernsteinsäure als geschmolzenes Material; und

(e) Einführen des geschmolzenen Materials in eine Flockenbildungsvorrichtung zur Herstellung eines Produkts in Flockenform.

6. Verfahren nach einem der vorhergehenden Ansprüche, worin das Reduktionsmittel Unterphosphorige Säure ist, die in einer Menge 0,5 bis 2,0 Gew.-%, bezogen auf das Gewicht des wäßrigen Gemisches, vorliegt.

7. Verfahren nach einem der Ansprüche 1 bis 5, worin das Reduktionsmittel Natriumhypophosphit ist, das in einer Menge von 1,0 bis 4,0 Gew.-%, bezogen auf das Gewicht das wäßrigen Gemisches, vorliegt.

8. Verfahren nach einem der vorhergehenden Ansprüche, worin 80 bis 95 Gew.-% des im wesentlichen dehydratisierten Gemisches in Form einer Mischung von Adipinsäure, Glutarsäure und Bernsteinsäure verdampft werden.

**Revendications**

1. Procédé pour récupérer un mélange semi-raffiné d'acides adipique, glutarique et succinique à partir d'un mélange aqueux dérivé d'un procédé pour l'oxydation du cyclohexane en acides dicarboxyliques, ledit mélange comprenant lesdits acides en même temps que des résidus de catalyseur et des impuretés, le procédé comprenant les étapes consistant à:

a) ajouter au mélange aqueux un agent réducteur choisi dans le groupe constitué de l'acide hypophosphoreux et de l'hypophosphite de sodium;

b) concentrer le mélange aqueous résultant par élimination de l'eau pour produire un mélange sensiblement déshydraté; et

c) amener le mélange sensiblement déshydrate à des moyens d'évaporation et en évaporer un mélange d'acides adipique, glutarique et succinique.

2. Procédé selon la revendication 1, dans lequel le mélange semi-raffiné récupéré d'acides adipique, glutarique et succinique peut réagir avec de l'éthylèneglycol et/ou du diéthylèneglycol pour produire un polyester de bas poids moléculaire ayant une couleur de pas plus d'environ 5 sur l'échelle Gardner.

3. Procédé selon la revendication 1 ou la revendication 2, dans lequel l'étape d'évaporation (c) est mise en oeuvre dans un évaporateur à film.

4. Procédé selon l'une quelconque des revendications précédentes, dans lequel l'étape de concentration (b) est mise en oeuvre dans un évaporateur à film.

5. Procédé selon l'une quelconque des revendications précédentes, comportant les étapes supplémentaires consistant à:

d) condenser le mélange d'acides adipique, glutarique et succinique évaporé des moyens d'évaporation dans l'étape (c) en tant que matière fondue; et

e) amener la matière fondue dans un appareil de formation de flocons pour produire un produit en forme de flocons.

6. Procédé selon l'une quelconque des revendications précédentes, dans lequel l'agent réducteur est de l'acide hypophosphoreux présent en une quantité de 0,5 à 2,0% en poids du mélange aqueux.

7. Procédé selon l'une quelconque des revendications 1 à 5, dans lequel l'agent réducteur est de l'hypophosphite de sodium présent en une quantité de 1,0 à 4,0% en poids du mélange aqueux.

8. Procédé selon l'une quelconque des revendications précédentes, dans lequel 80 à 95% en poids du mélange sensiblement déshydraté sont évaporés sous forme d'un mélange d'acides adipique, glutarique et succinique.